# EUROPÄISCHE PATENTSCHRIFT

(11) **EP 2 218 427 B1**
(45) Veröffentlichungstag und Bekanntmachung des Hinweises auf die Patenterteilung: **21.03.2012**
(21) Anmeldenummer: 10164256.9
(22) Anmeldetag: 21.12.2006
(51) Int. Cl.: A61F 5/00

(54) **Verfahren zum Steuern eines implantierten Magenbandes**
Method for controlling an implantable gastric band
Procédé de commande d'un anneau gastrique réglable

(30) Priorität: 22.12.2005 AT 20582005
(43) Veröffentlichungstag der Anmeldung: 18.08.2010
(62) Teilanmeldung aus: 06817495.2
(73) Patentinhaber: Lechner, Wolfgang, 3441 Pixendorf (AT)
(72) Erfinder: Lechner, Wolfgang, 3441 Pixendorf (AT)
(74) Vertreter: Sonn & Partner Patentanwälte

(56) Entgegenhaltungen:
- WO-A1-01/12078
- WO-A1-2005/009305

## Beschreibung

Die Erfindung betrifft ein Verfahren zum Steuern eines implantierten Magenbandes mit einer Fluid-befüllten Kammer, wobei der Druck an der Magenwand gemessen wird und aufgrund des ermittelten Druckes die Menge des Fluids in der Kammer des Magenbandes eingestellt wird.

Steuerbare Magenbänder, bei welchen eine Zufuhr von Flüssigkeit in die Kammer bzw. Entleerung von Flüssigkeit aus der Kammer durch Anstechen einer subkutan fixierten Kammer, eines so genannten Ports, der mit der Kammer des Magenbandes über eine entsprechende Leitung verbunden ist, erreicht wird, werden von zahlreichen Firmen in prinzipiell gleicher Bauart angeboten. Bei einem derartigen Magenband handelt es sich um ein zur Restriktion der Nahrungsaufnahme und damit zur Gewichtsreduktion eingesetztes medizinisches Implantat, das um den obersten Magenteil herum geschlungen und verschlossen wird.

Die US 6 966 875 B1 beschreibt ein Magenband, welches gürtelförmig um den Magen herumgelegt und befestigt wird. Eine Einstellung der Einengung des Stomas ist rein mechanisch durch Einengung des Bandes möglich.

Die US 4 592 339 A beschreibt ein Magenband, bei dem an der dem Magen zugewandten Seite des Bandes eine Kammer angeordnet ist, die mit Flüssigkeit aufgefüllt werden kann. Dadurch ist die Steuerung der Stomaweite möglich. Über einen subkutan eingenähten Port, der über einen Schlauch mit der Kammer des Magenbandes verbunden ist, kann eine Flüssigkeitsfüllung und Entleerung des Systems durchgeführt werden.

Die WO 2004/014245 A1 beschreibt ein steuerbares Magenband, wobei die Verschiebung der Flüssigkeit von einem Port in die Kammer des Magenbandes ferngesteuert von außen vorgenommen werden kann.

Neueste Forschungen haben gezeigt, dass dieser Bandinnendruck sehr exakt die Peristaltik der Speiseröhre wiedergibt. An Hand dieser Druckdaten lässt sich eine sehr exakte Einstellung der optimalen Stomaweite durchführen. Die Druckkurven geben auch Auskunft darüber, wie lange und wie oft gegessen bzw. getrunken wird. Daher ist der Bandinnendruck sehr gut für verschiedenste Kontrollzwecke in Zusammenhang mit der Anwendung von Magenbändern geeignet (W. Lechner et al: In Vivo Band Manometry: a New Access to Band Adjustment. Obesity Surgery, 15, 1432-1436, 2005).

Die derzeit verwendeten Magenbänder bringen in der Mehrzahl der Fälle sehr gute Langzeitergebnisse hinsichtlich Gewichtsreduktion und Patientenzufriedenheit. Dennoch gibt es einige Probleme die besonders bei hoher Bandauffüllung in den Vordergrund treten. Viele Patienten berichten über die unangenehme Erscheinung des Speichel-Erbrechens bzw. Herauswürgens, v.a. beim flachen Liegen. Speisereste können lange oberhalb des Stomas in der Speiseröhre verbleiben, hier zu gären beginnen und dadurch neben einem unangenehmen Mundgeruch eine Schleimhautreizung mit entsprechenden Schmerzen hervorrufen. Eine auf Dauer bestehende zu hohe Engstellung des Bandes führt im Verlauf von Monaten zu einer motorischen Erschöpfung der Speiseröhre die letztendlich in einer massiven Ausdehnung der Speiseröhre endet. Die geschluckten Speisen werden nicht mehr durch peristaltische Kontraktionswellen durch das Stoma in den Magen befördert sondern durch die Schwerkraft der sich oberhalb des Bandes auftürmenden geschluckten Speisen. Einen derartigen Zustand gilt es unbedingt zu verhindern, da damit auch der Verlust des Völlegefühls einhergeht. Die Bandwirkung geht dadurch verloren, was dann zu einer Gewichtszunahme trotz liegendem hoch aufgefülltem Magenband führt. Darüber hinaus ergeben sich verschiedenste mit der langen Verweildauer von Nahrungsbestandteilen im Ösophagus einhergehender Probleme wie retrosternales Brennen bzw."Sodbrennens" und rezidivierende Aspiration von Nahrungsanteilen.

Die WO 2005/009305 A1 betrifft ein steuerbares Magenband, bei dem die oben genannten Probleme durch eine autoregulative Veränderung der Stomaweite bewältigt werden sollen. Sie gründen auf den Änderungen des Bandinnendruckes im Zusammenhang mit der Peristaltik der Speiseröhre während eines Essvorgangs. Der Bolus wird durch peristaltische Wellen durch das Magenband hindurch gedrückt. Dadurch wird ein Druckanstieg in der Kammer des Magenbandes verursacht, welcher zum Regeln des Druckes des Magenbandes herangezogen werden kann.

Die WO 01/12078 A1 zeigt ein Kontrollsystem der gegenständlichen Art, wobei die Druckwerte an der Magenwand erfasst werden und an eine Kontrolleinheit übermittelt werden. Bei Über- oder Unterschreitung von vordefinierten Werten des Druckes erfolgt eine entsprechende Änderung der Auffüllung des Magenbands. Die aktuellen Druckwerte an der Magenwand sind jedoch für eine unmittelbare Einstellung der Bandweite des Magenbands nicht geeignet.

Die US 6 475 136 B1 beschreibt ein steuerbares Magenband, bei welchem ein implantierter Drucksensor den Druck an der Magenwand ermittelt und zur Regelung der Bandeinstellung heranzieht.

Unter klinischen Bedingungen wurde der Druck im Magenband während eines Essvorgangs bereits erfasst. Für den Erhalt ausreichender Informationen müssen jedoch Messungen über zumindest eine halbe Stunde und dies in wiederkehrenden Zeitintervallen vorgenommen werden, was für den Patienten sehr zeitaufwändig und belastend ist. Darüber hinaus entstehen in der Regel nach der Aufnahme der Daten noch Wartezeiten bis die Daten vom behandelten Arzt ausgewertet und die zu setzenden Schritte definiert werden.

Alternativ oder zusätzlich dazu werden auch Röntgenuntersuchungen nach der Verabreichung eines Kontrastmittels vorgenommen, was wesentlich schneller als die in vivo Messung des Bandinnendruckes abläuft. Diese Messungen sind jedoch für den Patienten aufgrund der Röntgenstrahlung belastend und darüber hinaus sehr kostenaufwändig.

Die Aufgabe der vorliegenden Erfindung besteht daher in der Schaffung eines oben genannten Verfahrens zum Steuern eines implantierten Magenbandes, mit welchem eine einfache und für den Patienten wenig belastende Überwachung des Druckes im Magenband möglich wird. Die vorliegende Erfindung soll die bisher auftretenden Probleme bei Magenbändern ohne autoregulative Funktion beheben bzw. reduzieren.

Gelöst wird die erfindungsgemäße Aufgabe durch ein oben genanntes Verfahren zum Steuern eines implantierten Magenbandes mit einer Fluid-befüllten Kammer, wobei der Druck an der Magenwand gemessen wird und aufgrund des ermittelten Druckes die Menge des Fluids in der Kammer des Magenbandes eingestellt wird, wobei die Druckwerte kontinuierlich gemessen werden und aus den gemessenen Druckwerten die Dauer und die Geschwindigkeit eines Essvorganges ermittelt wird. Aufgrund der ermittelten Parameter über dem Essvorgang kann eine Optimierung der Bandeinstellung durch den Arzt oder automatisch erfolgen oder dem Patienten wichtige Information über sein Essverhalten geliefert werden.

Dabei werden vorzugsweise die Amplitude und Dauer der Anstiege der Druckwerte während eines Essvorgangs für einen Rückschluss auf eine Veränderung der motorischen Leistungen des Ösophagus ausgewertet. Die Amplitude und Dauer der Anstiege der Druckwerte sind wichtige Indikatoren für eine zu starke Belastung des Ösophagus.

Aufgrund der ermittelten und ausgewerteten Druckwerte kann die Einstellung des Fluiddruckes in der Kammer des Magenbandes entsprechend optimiert werden.

Ein System zur Kontrolle eines steuerbaren Magenbandes sieht zumindest einen Speicher zum Speichern der ermittelten Druckwerte und eine Einrichtung zur Verarbeitung der zeitlichen Verläufe der empfangenen Druckwerte vor. Durch ein solches System wird es möglich, den Druck an der Magenwand während bestimmter Ereignisse, vorzugsweise während des Essens oder kontinuierlich zu erfassen und an eine Kontrolleinheit drahtlos zu übertragen. Dabei kann die Übertragung der erfassten Druckwerte permanent erfolgen oder es kann eine bestimmte Anzahl von Daten zwischengespeichert und auf Anforderung an eine Kontrolleinheit drahtlos übertragen werden. Der Patient muss sich zur Erfassung der Druckwerte nicht in ambulante Behandlung begeben, sondern es werden die wichtigen Daten während des normalen Tagesablaufs oder sogar der Nachtstunden gesammelt. Über die vorzugsweise kontinuierliche Überwachung des Druckes im Magenband können die Probleme derzeit verwendeter Magenbänder ohne autoregulative Funktion behoben werden. Der Druck, mit welchem das Magenband bzw. die Fluid-gefüllte Kammer an den Magen drückt, spielt eine Schlüsselrolle beim so genannten Gastric Banding. Die Bandinnendrucke spiegeln das Ausmaß der Abflussbehinderung und insbesondere die Rückwirkung der Abflussbehinderung auf die Motorik der Speiseröhre wieder. In dem zumindest einen Speicher können die Werte zwischengespeichert und bei Aufforderung zur Auswertung übertragen werden. Auch kann ein solcher Speicher zum Zwischenspeichern der Druckwerte im Fall einer Betriebsstörung dienen, so dass keine wichtige Information verloren geht. Die Ergebnisse liefern dem Patienten oder dem behandelnden Arzt oder dergl. wichtige Informationen. Durch das System wird eine Kontrolle von physiologischen Parametern, welche in Zusammenhang mit "Gastric Banding" von Bedeutung sind, erzielt. Das Kontrollsystem ermöglicht eine einfache und für den Patienten wenig belastende Überwachung der motorischen Funktion der Speiseröhre.

In der Folge von Schluckakten kommt es zu Druckanstiegen im Magenband. Die peristaltische Welle befördert den Bissen durch das Band in den Magen. Bei der Stomapassage kommt es zum erwähnten Druckanstieg im Bandsystem. Die Amplitude des Druckanstieges im Magenband hängt von der Amplitude der peristaltischen Welle, dem Ausmaß der Abflussbehinderung und der Viskosität des Bissens ab. Passiert der Bissen nicht vollständig das Band und bleibt daher ein Teil desselben in der Speiseröhre zurück, dann entstehen sekundäre peristaltische Wellen, d.h. peristaltische Wellen die nicht durch einen Schluckakt ausgelöst werden sondern durch die Dehnung der Speiseröhre durch den zurückgebliebenen Bissen. Es treten solange sekundäre peristaltische Wellen auf bis die Speiseröhre vom Bissen gereinigt ist. Diese Vorgänge können anhand des Bandinnendruckes mitverfolgt werden. Durch ein vorzugsweise kontinuierliches Erfassen des Druckverlaufes im Bandinneren kann die motorische Funktion der Speiseröhre exakt überwacht werden. Eine zu enge Bandeinstellung führt dazu, dass Speisereste sehr lange oberhalb des Bandes verbleiben, fortlaufend eine sekundäre peristaltische Aktivität auslösen, was letztendlich zur motorischen Erschöpfung der Speiseröhre führt. Genau das kann durch ein Druckmonitoring verhindert werden. Der Arzt kann bei der Bandengstellung bis an die Grenze des Verträglichen gehen und damit den maximalen Therapie-Effekt erzielen ohne eine Gefährdung der motorischen Aktivität der Speiseröhre in Kauf nehmen zu müssen. Durch das Druckmonitoring kann eine optimale Magenbandeinstellung aber auch eine Selbstkontrolle des Patienten hinsichtlich des Essverhaltens erreicht werden.

Die Übertragungseinrichtung ist vorzugsweise durch einen Hochfrequenzsender und die Empfangseinrichtung durch einen Hochfrequenzempfänger gebildet. Die Leistung des mitimplantierten Hochfrequenzsenders ist für die Erzielung relativ niedriger Reichweiten relativ gering, wodurch der Stromverbrauch ebenfalls minimal ist und somit die Lebensdauer des Implantats erhöht ist.

Ebenso ist es möglich, den Hochfrequenzsender durch einen passiven Sender zu bilden und die für die Übertragung der erfassten Druckwerte notwendige Energie von außen induktiv in das System einzukoppeln. Derartige passive Systeme sind bei anderen Implantaten bereits bekannt.

Um eine Bidirektionalität der Datenübertragung zwischen Kontrolleinheit und implantierten Magenband zu erzielen, kann die Übertragungseinrichtung im Magenband und die Empfangseinrichtung in der Kontrolleinheit durch einen Hochfrequenztransponder, welcher sowohl Sende- als auch Empfangsfunktion übernimmt, gebildet sein.

Gemäß einem weiteren Merkmal des Kontrollsystems ist zumindest ein Drucksensor durch einen piezoelektrischen Sensor gebildet. Der Sensor kann an einer beliebigen Stelle im Magenband angeordnet sein, welche sich zur Messung des Druckes, welcher auf die Magenwand wirkt, eignet, also beispielsweise in der Kammer oder an der stomaseitig angeordneten Kammerwand oder dgl.

Wenn ein Speicher zum Speichern der ermittelten Druckwerte im Magenband vorgesehen ist, können die Werte zwischengespeichert und bei Aufforderung an die Kontrolleinheit zur Auswertung übertragen werden. Auch kann ein solcher Speicher im Magenband zum Zwischenspeichern der Druckwerte im Falle einer Betriebsstörung der Kontrolleinheit dienen, so dass keine wichtige Information verloren geht.

Ebenso kann ein Speicher zum Speichern der ermittelten Daten einerseits und allfälliger Berechnungsformeln oder Programmen andererseits in der Kontrolleinheit vorgesehen sein. Weiters ist in der Kontrolleinheit vorzugsweise eine Anzeige vorgesehen, über die dem Patienten die Daten bzw. daraus abgeleitete Daten übermittelt werden können. Dabei kann die Anzeige einerseits aus einfachen Leuchtmitteln bestehen, über die gewisse Zustände optisch dargestellt werden oder auch durch Ziffernanzeigen zur Darstellung der Druckwerte oder daraus abgeleiteter Werte bis hin zu Bildschirmen, über die der Verlauf des Druckes über der Zeit grafisch dargestellt werden kann.

Die Einrichtung zur Verarbeitung der empfangenen Druckwerte ist vorzugsweise durch einen Mikroprozessor gebildet.

Um eine zeitliche Zuordnung der Daten zu erzielen, kann in der Kontrolleinheit ein Zeitmodul vorgesehen sein, wie es bei Mikroprozessoranwendungen üblicherweise der Fall ist.

Zur Inbetriebnahme der Kontrolleinheit oder auch zur Umschaltung von Betriebszuständen kann in der Kontrolleinheit zumindest ein Bedienungselement vorgesehen sein.

Um dem Patienten oder dem behandelnden Arzt das Eintreten bestimmter Zustände oder dgl. anzuzeigen, kann in der Kontrolleinheit ein Signalgeber vorgesehen sein.

Ein solcher Signalgeber kann beispielsweise durch einen Lautsprecher oder einen Schwingungserzeuger gebildet sein. Letzterer hat den Vorteil, dass ähnlich wie beim Vibrieren eines Mobiltelefons die Umgebung nicht auf das Signal des Signalgebers aufmerksam gemacht wird.

Schließlich verfügt die Kontrolleinheit vorzugsweise über eine Schnittstelle, um die Daten beispielsweise an einen Computer oder dgl. zu übertragen. Auf diese Weise ist es auch möglich, dass der Patient die Daten zur Auswertung an die Klinik oder den behandelnden Arzt beispielsweise über Internet oder am Telefonnetz übermittelt und für eine Befundung nicht an die Klinik oder zum behandelnden Arzt muss.

Vorteilhafterweise ist das Gehäuse der Kontrolleinheit in Form einer Armbanduhr mit einem entsprechenden Uhrband gebildet. Auf diese Weise kann der Patient mit dem implantierten Magenband ständig die Kontrolleinheit in bequemer Art und Weise bei sich tragen und wird somit ständig über den Zustand des Magenbandes informiert. Ebenso können die Daten über einen bestimmten Zeitraum, beispielsweise 24 Stunden, gespeichert werden und danach die Kontrolleinheit an eine Auswerteeinrichtung, beispielsweise beim behandelnden Arzt, übermittelt werden, wo eine Analyse der Daten stattfindet.

Weiters kann eine Einrichtung zur Einbringung oder Absaugung des Fluids in oder aus der Kammer des Magenbandes vorgesehen sein, so dass aufgrund der erfassten und ausgewerteten Druckwerte der Druck in der Kammer des Magenbandes optimierbar ist. Auf diese Weise kann ein automatisches oder halbautomatisches System geschaffen werden, welches aufgrund der erfassten Druckwerte das Magenband immer optimal einstellt. Die Einrichtung zur Einbringung oder Absaugung des Fluids in oder aus der Kammer kann dabei ebenfalls durch ein Implantat gebildet sein, welches die entsprechende Information drahtlos von der Kontrolleinheit übermittelt bekommt.

Die Einrichtung zur Einbringung oder Absaugung des Fluids kann durch eine in der Verbindung zwischen der Kammer und der zweiten Kammer des Magenbandes angeordneten Pumpe gebildet sein, welche das Fluid aus der Kammer in die zweite Kammer oder umgekehrt befördern kann.

Zusätzlich kann eine wichtige Information über das Essverhalten des Patienten mit Hilfe eines Sensors zur Messung der Schluckaktivität geschaffen werden, welcher Sensor mit einer Einrichtung zur drahtlosen Übertragung der erfassten Sensorwerte an die Kontrolleinheit verbunden ist. Ein derartiger Schlucksensor kann beispielsweise durch ein Mikrofon oder einen Druckwandler, der an den Hals des Patienten aufgeklebt wird, gebildet sein.

Gemäß einem weiteren Merkmal des Kontrollsystems ist eine Leitung zur Verbindung der Kontrolleinheit mit einer Kommunikationseinrichtung, insbesondere einem Computer oder einem Telefon, vorgesehen. Auf diese Weise kann über die Schnittstelle der Kontrolleinheit und die besagte Leitung eine Datenverbindung zu einer Kommunikationseinrichtung und von dort zu weiteren Kommunikationseinrichtungen, beispielsweise im Internet oder einem Telefonnetz, hergestellt werden.

Die zweite Kammer des Magenbandes ist üblicherweise bei den gegenständlichen Magenbändern ohne autoregulative Funktion, üblicherweise durch einen subkutan anzuordnenden Port gebildet, über welchen mit Hilfe einer Subkutanspritze das Fluid in die Kammer des Magenbandes eingebracht oder aus dieser abgesaugt werden kann.

Die beiden Kammern des steuerbaren Magenbandes bzw. die Kammer und der Port, sind vorzugsweise über eine Leitung miteinander verbunden, wobei in der Leitung zumindest eine Einrichtung zur Steuerung des Durchflusses des Fluids angeordnet sein kann. Diese Durchflusssteuerungseinrichtung kann beispielsweise durch ein Ventil oder eine Pumpe gebildet sein, wodurch eine automatische Regelung des Druckes in der Kammer des Magenbandes und somit auf die Magenwand aufgrund der gemessenen Druckwerte ermöglicht wird.

Vorteilhafterweise ist die Verarbeitungseinrichtung zur Ermittlung der Dauer und Geschwindigkeit eines Essvorganges ausgebildet. Aufgrund der ermittelten Parameter über den Essvorgang, kann eine Optimierung der Bandeinstellung durch den Arzt oder automatisch erfolgen oder dem Patienten wichtige Information über sein Essverhalten geliefert werden.

Dabei ist die Verarbeitungseinrichtung vorzugsweise zur Auswertung der Amplitude und Dauer der ermittelten Druckwerte während eines Essvorganges ausgebildet.

Die Amplitude und Dauer der Anstiege der Druckwerte sind wichtige Indikatoren für eine zu starke Belastung des Ösophagus.

Die Erfindung wird anhand der folgenden Abbildungen näher erläutert. Darin zeigen:
- Fig. 1: eine schematische Darstellung eines Kontrollsystems beste- hend aus dem Magenband mit dem Sensor und der Kontrollein- heit;
- Fig. 2: eine schematische Darstellung eines implantierten Magen- bandes mit einer Kontrolleinheit in Form einer Armbanduhr;
- Fig. 3: eine schematische Darstellung der Datenübertragung von ei- ner Kontrolleinheit beispielsweise zu einem Computer;
- Fig. 4: den zeitlichen Verlauf des erfassten Druckes im Magenband während mehrerer Schluckvorgänge bei verschiedenen Füllun- gen der Kammer des Magenbandes; und
- Fig. 5: den zeitlichen Verlauf des Druckes im Magenband während eines Essvorganges.

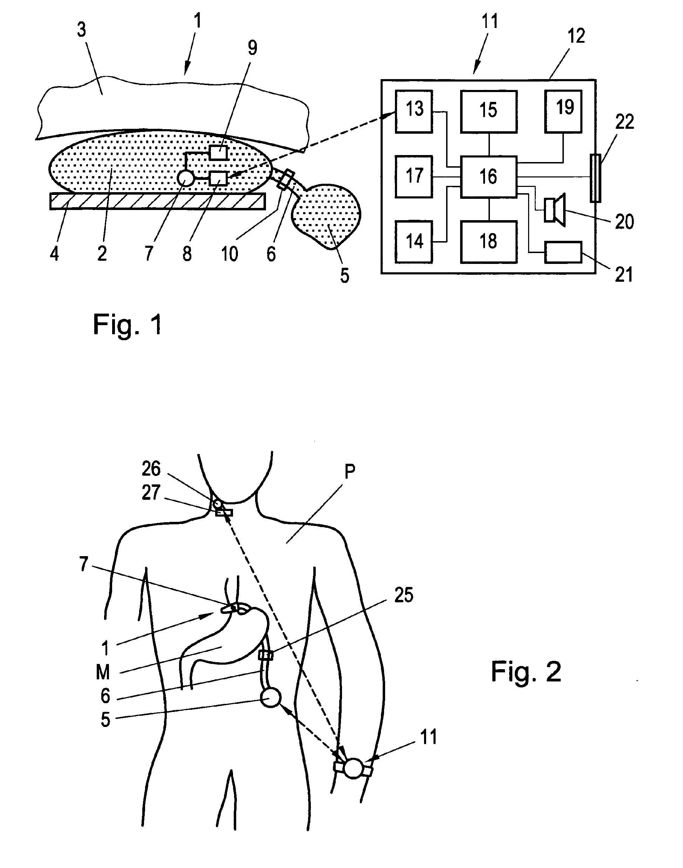

Fig. 1 zeigt schematisch ein Magenband 1 zusammen mit einer Kontrolleinheit 11, mit Hilfe welcher der Druck des Magenbandes 1 überwacht werden kann. Das Magenband 1 besitzt einen nicht dehnbaren Rücken 4. An der der Magenwand 3 zugewandten Seite des Rückens 4 befindet sich zumindest eine Kammer 2, welche mit einer Flüssigkeit bzw. einem Fluid befüllbar ist. Die Kammer 2 ist mit einer zweiten Kammer 5 über eine Leitung 6 verbunden. Durch Verschiebung der Flüssigkeit zwischen den Kammern 2 und 5 kann die Einengung des um den Mageneingang gelegten Magenbandes 1 und somit der Druck auf den Magen verändert werden. Die zweite Kammer 5 kann als subkutan im Unterbauch angeordneter so genannter Port ausgebildet sein (siehe Fig. 2), über welchen von außen mittels einer Subkutanspritze Flüssigkeit zu- oder abgeführt und somit die Flüssigkeitsmenge und in der Folge der Druck in der Kammer 2 verändert werden kann. Zur Steuerung des Durchflusses der Flüssigkeit bzw. des Fluids von der einen Kammer 2 zur anderen Kammer 5 bzw. umgekehrt kann auch in der Verbindungsleitung 6 eine entsprechende Einrichtung 10 zur Steuerung des Durchflusses der Flüssigkeit vorgesehen sein. Diese Steuerungseinrichtung 10 kann beispielsweise durch ein Ventil oder eine Pumpe gebildet sein (nicht dargestellt). In der Kammer 2 bzw. an einer anderen geeigneten Stelle, wie zum Beispiel in der zweiten Kammer 5, ist zumindest ein Sensor 7 zur Messung des auf die Magenwand 3 wirkenden Druckes bzw. einer dazu proportionalen Größe vorgesehen, welcher Sensor 7 mit einer Einrichtung 8 zur drahtlosen Übertragung der erfassten Druckwerte an eine Kontrolleinheit 11 verbunden ist. Zum Speichern bzw. Zwischenspeichern der vom Drucksensor 7 erfassten Druckwerte kann ein Speicher 9 vorgesehen sein. Die implantierte Übertragungseinrichtung 8 kann durch eine Batterie mit elektrischer Energie versorgt werden, oder vollständig passiv aufgebaut sein, wobei die für die Übertragung der Daten notwendige Energie von außen, insbesondere vom Empfänger der Kontrolleinheit 11, eingebracht werden würde. Um eine Datenübertragung nicht nur vom Magenband 1 zur Kontrolleinheit 11 sondern auch umgekehrt zuzulassen, kann die Übertragungseinrichtung 8 auch durch einen Transponder gebildet sein.

Die Kontrolleinheit 11 beinhaltet eine Einrichtung 13 zum Empfang der übertragenen Druckdaten des Sensors 7 des Magenbandes 1, die ebenfalls als Transponder ausgebildet sein kann. Neben einem Speicher 14 zum Speichern der übertragenen Daten aber auch von Programmen ist vorzugsweise eine Anzeige 15 im Gehäuse 12 der Kontrolleinheit 11 angeordnet. Über eine Einrichtung 16 werden die empfangenen Druckwerte verarbeitet. Diese Verarbeitungseinrichtung 16 ist vorzugsweise durch einen Mikroprozessor, Mikrokontroller oder dgl. gebildet. Um die erfassten und übermittelten Daten zeitlich zuordnen zu können, kann ein Zeitmodul 17 mit der Verarbeitungseinrichtung 16 verbunden sein. Weiters kann für die Inbetriebnahme der Kontrolleinheit 11 aber auch zum Umschalten von Betriebszuständen zumindest ein Bedienungselement 18 vorgesehen sein.

Um den Patienten aber auch den Arzt über das Eintreten bestimmter Zustände zu informieren, kann ein Signalgeber, beispielsweise ein Lautsprecher 20 oder ein Schwingungserzeuger 21 vorgesehen sein. Die Komponenten der Kontrolleinheit 11 werden von einer Spannungsversorgung 19, welche vorzugsweise durch eine wiederaufladbare Batterie gebildet ist, mit elektrischer Energie versorgt.

Um die im Speicher 14 gespeicherten Daten beispielsweise an einen Computer übertragen zu können, kann eine Schnittstelle 22 vorgesehen sein. Über eine derartige Schnittstelle 22 können auch Daten in die Verarbeitungseinrichtung 16 oder in den Speicher 14 geschrieben werden. Die Schnittstelle 22 kann beispielsweise durch verschiedene standardisierte Schnittstellen gebildet sein. Dabei kommen sowohl leitungsgebundene als auch drahtlose (z.B. Infrarot- oder Hochfrequenz-) Schnittstellen zur Anwendung.

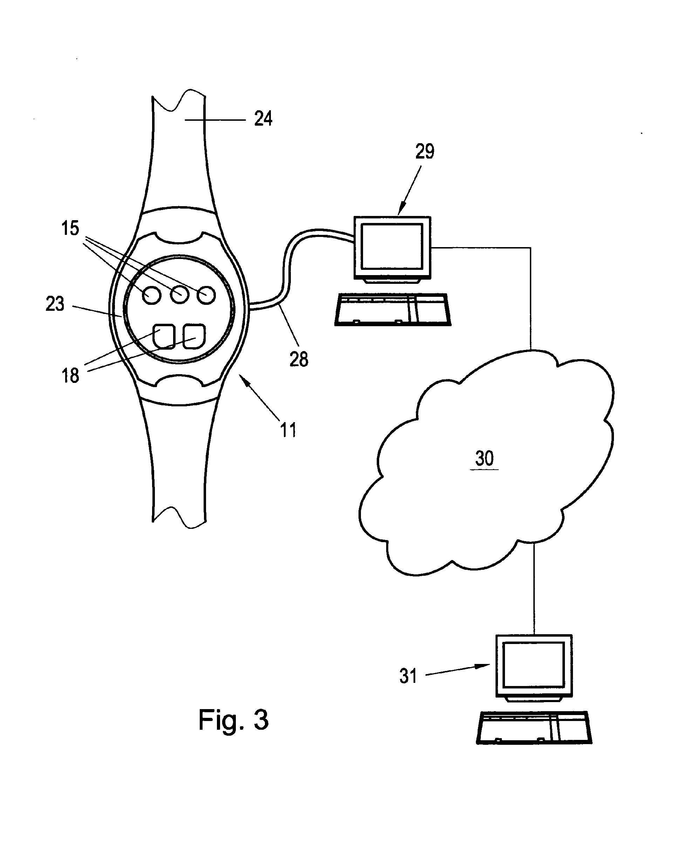

Fig. 2 zeigt schematisch eine Anwendung des erfindungsgemäßen Verfahrens bei einem Magenband 1 mit zumindest einem Drucksensor 7, bei welchem die zweite Kammer 5 durch einen so genannten subkutanen Port gebildet ist, der mit der Kammer 2 des Magenbandes 1 über eine Leitung 6 verbunden ist, in welcher beispielsweise eine Pumpe 25 angeordnet sein kann, welche den Flüssigkeitstransport von der Kammer 2 zur Kammer 5 oder umgekehrt steuert. Das Magenband 1 umschließt den Eingang des Magens M des Patienten P. Die Daten des Drucksensors 7 werden an die Kontrolleinheit 11, welche im dargestellten Beispiel in Form einer Armbanduhr mit einem entsprechenden Uhrband (s. Fig. 3) gebildet ist, übermittelt, wo sie verarbeitet und gespeichert bzw. angezeigt werden. Zusätzlich zum Drucksensor 7 im Magenband 1 kann ein Sensor 26 zur Messung der Schluckaktivität des Patienten P mit einer entsprechenden Einrichtung 27 zur drahtlosen Übertragung verbunden sein und können diese Daten ebenfalls drahtlos an die Kontrolleinheit 11 übermittelt werden. Für den Patienten P dient die Kontrolleinheit 11 als Biofeedback-Instrument, welches sein Essverhalten wiederspiegelt. Neben der Dauer der Nahrungsaufnahme können auch andere Faktoren, wie die Geschwindigkeit der Nahrungsaufnahme und das Ausmaß des Kauens ermittelt und angezeigt werden. Eine große Essgeschwindigkeit führt genauso wie schlechtes Kauen zu höheren Druckanstiegen im Magenband. Damit wird das Druckmonitoring für den Patienten zu einem Instrument der Selbstkontrolle hinsichtlich des Essverhaltens. Darüber hinaus können Störungen des Systems, z.B. durch Flüssigkeitsverlust wegen Undichtwerden des Magenbandes 1 sofort erkannt und dem Patienten P rückgemeldet werden.

Für den Träger des Magenbandes 1 ist eine Änderung der Esstechnik erforderlich, um unangenehme Sensationen bzw. ein Erbrechen zu verhindern. Es ist ein langsames Essen mit gutem Kauen sowie die Einhaltung von entsprechenden Pausen zwischen den einzelnen Bissen erforderlich. Der hochgradig adipöse Patient mit fast immer entsprechend massiver Essstörung tut sich oft schwer auf diese richtige Esstechnik umzustellen. Eine fortwährend beibehaltene falsche Esstechnik kann den Langzeit-Therapieerfolg mit dem Magenband beeinträchtigen und zu einer schweren motorischen Störung der Ösophagusperistaltik bzw. einer massiven Ausdehnung der Speiseröhre führen. Daher erscheint es als sehr vorteilhaft, dem Patienten mit Hilfe der Kontrolleinheit 11 eine ständige Rückmeldung zu geben, ob er die Nahrungsaufnahme in der richtigen Art und Weise durchführt und ob er zu lange isst.

Die Kontrolleinheit 11 bereitet die Druckdaten in einer für den Patienten unmittelbar einsichtigen Form auf und gibt ihm sofortige Rückmeldungen hinsichtlich seines Essverhaltens sowie Warnhinweise bei nicht einwandfreiem Funktionieren des Magenbandes 1 sowie bei Zeichen der muskulären Erschöpfung der Speiseröhre. Er wird dann auf die Notwendigkeit einer sofortigen ärztlichen Kontrolle hingewiesen. Die Rückmeldungen an den Patienten P können über optische Informationen auf einer Anzeige 15 oder durch akustische oder taktile Signalgeber übermittelt werden (s. Fig. 1).

Fig. 3 zeigt schematisch ein Blockschaltbild über die Datenfernübertragung von der Kontrolleinheit 11 beispielsweise zu einem Computer 31. In diesem Fall wird die Kontrolleinheit 11, vorzugsweise über die Schnittstelle 22 (s. Fig. 1), mit einer entsprechenden Leitung 28 mit einer Kommunikationseinrichtung, beispielsweise einem Computer 29, verbunden. Auf diese Weise können die in der Kontrolleinheit 11 enthaltenen Daten an den Computer 29 und von diesem über ein Datennetz 30, insbesondere das Internet, zu einem weiteren Computer 31 übermittelt werden. Auf diese Weise kann der Patient die Daten seiner Kontrolleinheit 11 in bequemer und zeitsparender Weise an einen Computer 31 der Klinik oder des behandelnden Arztes überweisen, wo diese entsprechend ausgewertet werden.

Die Optimierung der Bandeinstellung ist ein wichtiger Punkt, um einerseits einen maximalen Therapieerfolg zu erreichen, und um andererseits Schädigungen der Speiseröhren durch ein zu langes Verweilen von Speisen im Ösophagus zu verhindern (siehe oben). Das Zurückbleiben von Anteilen des Speisebreis in der Speiseröhre regt das Auftreten von sekundären peristaltischen Wellen an. Diese sekundäre Peristaltik versucht den Bolus weiter zu befördern und die Speiseröhre zu reinigen. Gelingt dies nicht und bleiben Speisereste ständig oberhalb des Magenbandes liegen, führt dies im Verlauf von Tagen und Wochen einerseits zu einer Schleimhautentzündung mit entsprechenden Schmerzen, andererseits zu einer motorischen Erschöpfung und zunehmenden Ausdehnung der Speiseröhre.

Für den Arzt ist daher die Kontrolle der von ihm durchgeführten Bandeinstellung von großer Bedeutung. Die Qualität der Einstellung erweist sich jedoch erst im Tagesablauf im Rahmen der Nahrungsaufnahmen und der Schlafphasen. Dann zeigt sich erst, ob die gewählte Bandeinstellung zu einem zu langen postprandialen Verweilen von Nahrungsresten in der Speiseröhre führt bzw. während der Nachtruhe zu einem schlechten Abfließen des Speichels, etc. Daher empfiehlt sich zum Zwecke der optimalen Bandadjustierung die Aufzeichnung der Druckdaten über einen eingeschränkten Zeitraum, etwa über 24 Stunden. An Hand dieser Daten, die nunmehr sehr bequem erfasst und übertragen werden können, kann dann eine sehr exakte Nachadjustierung des Bandes durch den Arzt vorgenommen werden.

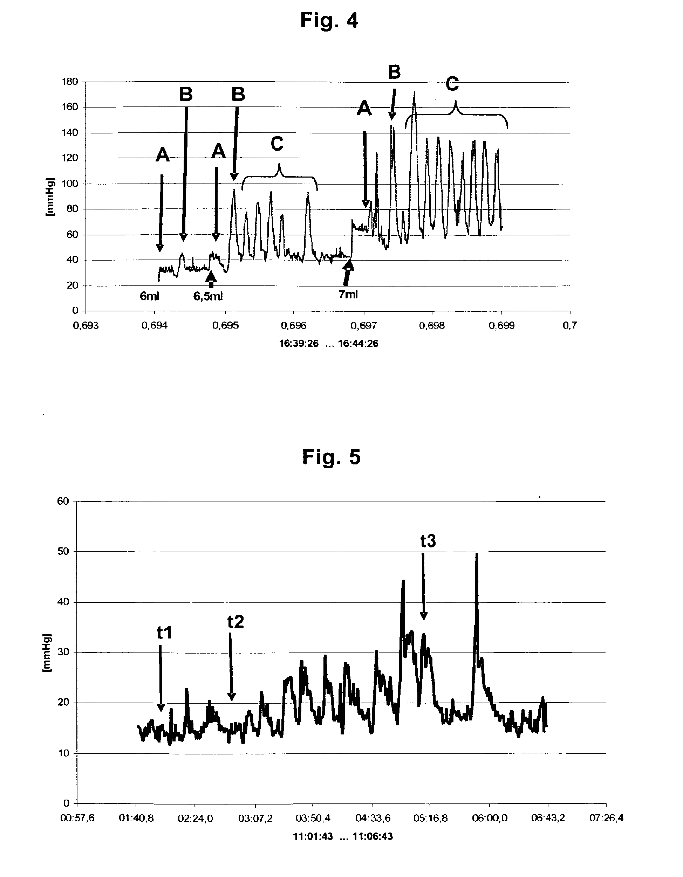

Fig. 4 zeigt den zeitlichen Verlauf des Druckes im Magenband während mehrerer Schluckvorgänge von jeweils 15 ml Wasser bei drei verschiedenen Bandfüllungen, nämlich 6 ml, 6,5 ml und 7 ml. Dieses Zeit-Druckdiagramm zeigt die Abhängigkeit der sekundären Peristaltik vom Ausmaß der Engstellung des Bandes und damit der Abflussbehinderung. Bei jeder Bandfüllung hat der Patient einen 15 ml Wasserschluck durchzuführen (Pfeil A). Bei 6 ml Bandfüllung zeigt sich nur ein einzelner Druckanstieg im Magenband. Dieser Druckanstieg entspricht der durch den Schluckakt ausgelösten primären peristaltischen Welle (Pfeil B). Bei 6,5 ml Adjustierung des Bandes folgen auf die primäre peristaltische Welle fünf sekundäre peristaltische Wellen (Bereich C) (nicht durch einen Schluckakt ausgelöst). Dies weist auf die bereits sehr ausgeprägte Abflussbehinderung hin. Mit 7 ml Füllvolumen ist das Band nicht mehr durchgängig. Dies drückt sich in der fortgesetzten sekundären Peristaltik (Bereich C) im Anschluss an den 15 ml Wasser-Schluckakt aus. Die optimale Bandfüllung liegt in diesem Fall im Bereich zwischen 6 und 6,5 ml.

Fig. 5 zeigt schließlich den zeitlichen Verlauf des erfassten Druckes in einem Magenband während eines aus Suppe und Dillkartoffeln zusammengesetzten Essens. Zum Zeitpunkt t1 beginnt der Proband mit dem Essen der Suppe, zum Zeitpunkt t2 mit dem Essen der Kartoffel. Der Pfeil mit der Bezeichnung t3 markiert die Beendigung des Essens. Die im Bandinneren abgeleiteten Druckwellen geben die Dauer des Essens wieder. Nach Beendigung des Essens können in Abhängigkeit vom Ausmaß der Abflussbehinderung im Bandinneren noch für unterschiedliche Zeit Druckwellen abgeleitet werden.

Abschließend sei erwähnt, dass aus den zeitlichen Druckverläufen durch die anatomische Nähe des Sensors 7 zum Herzen auch die Herzkontraktionen erkennen und damit den Herzrhythmus ableiten kann.

## Patentansprüche

1. Verfahren zum Steuern eines implantierten Magenbandes (1) mit einer Fluid-befüllten Kammer (2), wobei der Druck an der Magenwand (3) gemessen wird und aufgrund des ermittelten Druckes die Menge des Fluids in der Kammer (2) des Magenbandes (1) eingestellt wird, wobei die Druckwerte kontinuierlich gemessen werden und aus den gemessenen Druckwerten die Dauer eines Essvorganges ermittelt wird, **dadurch gekennzeichnet, dass** aus den gemessenen Druckwerten auch die Geschwindigkeit des Essvorganges ermittelt wird.

2. Verfahren nach Anspruch 1, **dadurch gekennzeichnet, dass** die Amplitude und Dauer der Anstiege der Druckwerte während eines Essvorganges für einen Rückschluss auf eine Veränderung der motorischen Leistungen des Ösophagus ausgewertet werden.

3. Verfahren nach Anspruch 1 oder 2, **dadurch gekennzeichnet, dass** aufgrund der ermittelten und ausgewerteten Druckwerte die Einstellung des Fluiddruckes in der Kammer des Magenbandes (1) optimiert wird.

## Claims

1. A method for controlling an implanted gastric band (1) with a fluid-filled chamber (2), wherein the pressure at the wall of the stomach (3) is measured and the amount of fluid in the chamber (2) of the gastric band (1) is adjusted on the basis of the pressure determined, wherein the pressure values are measured continuously, and wherein the duration of an eating process is determined from the pressure values measured ,
**characterized in that** the rate of the eating process is also determined from the pressure values measured.

2. The method according to claim 1, **characterized in that** the amplitude and the duration of the rises of the pressure values during an eating process are evaluated for an indication of a modification of the motoric activity of the esophagus.

3. The method according to claims 1 or 2, **characterized in that** the adjustment of the fluid pressure in the chamber of the gastric band (1) is optimized on the basis of the pressure values that have been determined and evaluated.

## Revendications

1. Procédé de commande d'un anneau gastrique implanté (1) comprenant une chambre (2) remplie d'un fluide, dans lequel on mesure la pression au niveau de la paroi gastrique (3) et en se basant sur la pression déterminée on règle la quantité de fluide dans la chambre (2) de l'anneau gastrique (1), dans lequel on mesure en continu les valeurs de pression et on détermine à partir des valeurs de pression mesurées la durée d'un processus de nutrition,
**caractérisé en ce que** l'on détermine également à partir des valeurs de pression mesurées la vitesse du processus de nutrition.

2. Procédé selon la revendication 1, **caractérisé en ce qu'**on évalue l'amplitude et la durée des montées des valeurs de pression pendant un processus de nutrition pour une conclusion quant à une modification des performances motrices de l'oesophage.

3. Procédé selon la revendication 1 ou 2, **caractérisé en ce que** l'on optimise le réglage de la pression du fluide dans la chambre de l'anneau gastrique (1) en se basant sur les valeurs de pression déterminées et évaluées.
